# NEW EUROPEAN PATENT SPECIFICATION

(11) **EP 2 105 101 B2**
(45) Date of publication and mention of the opposition decision: **11.09.2013**
(45) Mention of the grant of the patent: 29.09.2010
(21) Application number: 08005981.9
(22) Date of filing: 28.03.2008
(51) Int. Cl.: A61B 17/70

(54) **Bone anchoring device**
Knochenverankerungsvorrichtung
Dispositif d'ancrage d'os

(43) Date of publication of application: 30.09.2009
(73) Proprietor: BIEDERMANN MOTECH GmbH, 78054 VS-Schwenningen (DE)
(72) Inventor: Biedermann, Lutz, 78048 VS-Villingen (DE)
(74) Representative: Hofer, Dorothea

(56) References cited:
- EP-A- 1 800 613
- WO-A-2006/073593
- WO-A-2006/096241
- WO-A-2007/070757
- WO-A-2007/122494
- WO-A2-2007/089984
- US-A1- 2007 055 244

## Description

The invention relates to a bone anchoring device for the dynamic stabilization of bones or vertebrae.

EP 1 759 646 A1 discloses a spinal implant for the dynamic stabilization of vertebrae using a flexible rod which is clamped in the receiving part of a pedicle screw by means of a filling piece and a clamping element. The pedicle screw is a so-called monoaxial pedicle screw.

EP 1 795 134 A1 discloses a polyaxial screw for use with a flexible rod. A pressure element and a filling piece clamp the flexible rod between each other so that a flow of the material of the flexible rod in direction along the longitudinal axis of the rod is minimized.

The known bone anchoring devices are specifically adapted to be used with a flexible rod.

Document WO 2006/096241 A2 discloses a vertebral stabilization using a flexible rod. The rod connects two tulips each holding a bone anchor. The rod has a flexible section provided by a helical recess formed as a through-cut in a cylindrical wall enclosing an inner bore. A set screw is provided to fixate the rod within the tulip.

For this purpose, the rod has at its one end beyond the flexible section a coupling formed by a thin post. A sleeve can be clamped around this post. This sleeve is fitted into the tulip, and the set screw is used to compress the sleeve onto the post. The sleeve is therefore with a slot.

A bone anchoring device according to the preamble of claim 1 is disclosed in WO 2007/122494 A2.

It is the object of the invention to provide a bone anchoring device for the dynamic stabilization of bones or vertebrae which uses a flexible rod and which has a simplified design.

The object is solved by a bone anchoring device according to claim 1. Further developments of the invention are given in the dependent claims.

The bone anchoring device allows the use of a flexible rod together with known monoaxial or polyaxial screws which must not be specifically adapted to clamp the flexible rod. The clamping of the flexible rod is achieved by means of known fixation elements, such as, for example, set screws.

Known monoaxial or polyaxial screws which are used with metallic rods can be upgraded so that they can be used with flexible rods.

The sleeve which clamps the flexible rod can have different dimensions in order to allow the use of rods of different diameter with one and the same receiving part.

Further features and advantages of the invention will become apparent from the detailed description of embodiments in conjunction with the accompanying drawings. In the drawings:
- Fig. 1: shows a perspective elevational view of a bone an- choring device according to a first embodiment.
- Fig. 2: shows the bone anchoring device of Fig. 1 in an as- sembled state.
- Fig. 3: shows a sectional view of the bone anchoring device of Fig. 2 in an assembled state, the section being taken perpendicular to the longitudinal axis of the rod.
- Fig. 4: shows a perspective view of the sleeve.
- Fig. 5: shows a sectional view of the sleeve, the section being taken along line A-A.
- Fig. 6: shows a perspective elevational view of the bone an- choring device according to a second embodiment.
- Fig. 7: shows a perspective view of the bone anchoring de- vice of Fig. 6 in an assembled state.
- Fig. 8: shows a sectional view of the bone anchoring device of Fig. 7, the section being taken in a plane per- pendicular to the rod axis.

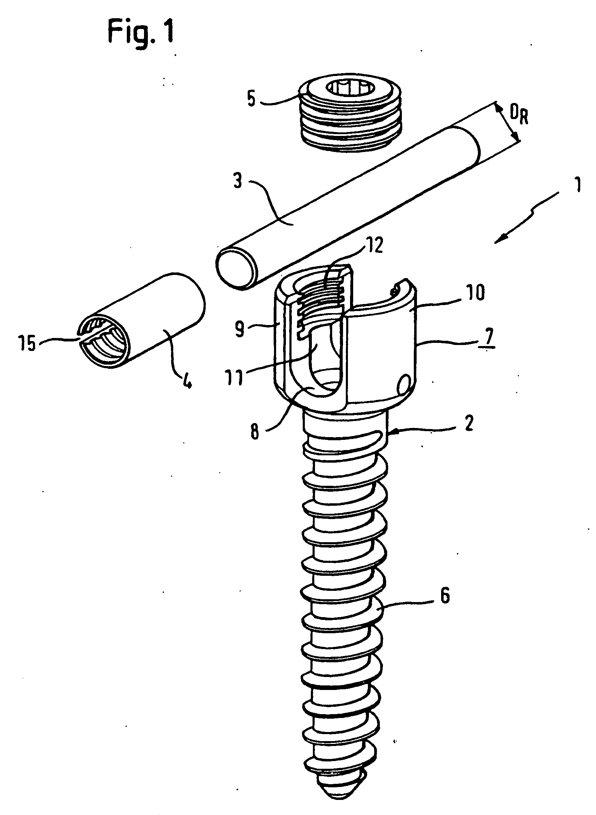

With reference to the drawings, Figs. 1 to 3 show a first embodiment of the bone anchoring device 1 which is comprised of a monoaxial pedicle screw with rod. The bone anchoring device 1 comprises a bone anchoring element 2, a flexible rod 3, a sleeve 4 and a fixation element 5. The bone anchoring element 2 comprises a threaded shaft 6 for anchoring in the bone or in a vertebra and a receiving part 7, which is essentially cylindrical in shape and which has a coaxial bore 11 and an essentially U-shaped recess 8 forming two free legs 9 and 10. An inner thread 12 is provided at the free legs 9, 10. In the first embodiment the receiving part 7 and the threaded shaft 8 are made of one piece. The angular position of the threaded shaft 8 relative to the receiving part 7 is fixed , in the example it is coaxial, so that a monoaxial screw is provided. The fixation element 5 is a set screw which can be screwed in between the legs 9, 10.

The rod 3 has a generally circular cross-section and is partially or fully flexible. The rod is at least partly made of an elastomer material. The material can be a polymer on the basis of polyurethane or polycarbonateurethane (PCU).

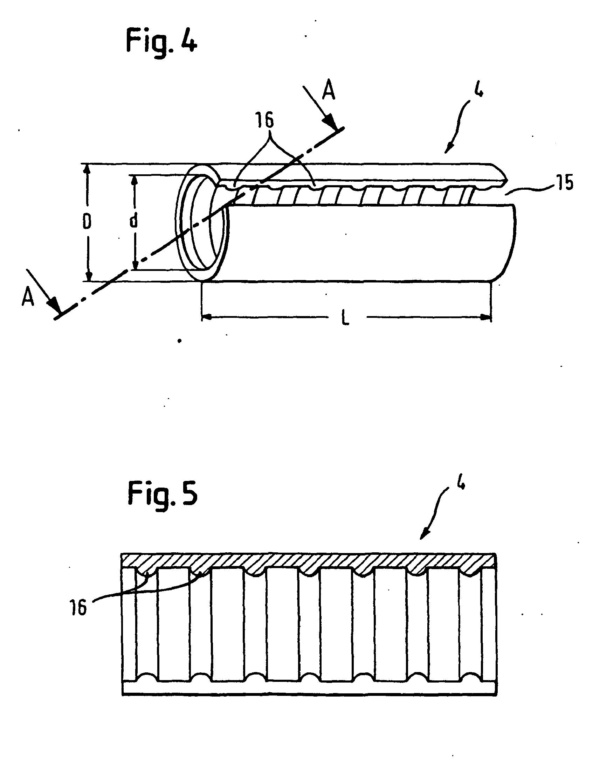

As can be seen particularly in Figs. 1 to 4, the sleeve 4 is substantially cylindrical with an outer diameter D which is sized to fit into the U-shaped recess 8 of the receiving part 7. The inner diameter d is slightly larger than the diameter D_{R} of the rod 3 so that the sleeve can be placed onto the rod. Alternatively the size of inner diameter d of the rod can be equal or slightly smaller than the diameter of the rod so that the sleeve can be placed onto the rod in a pre-tensioned manner. The length L of the sleeve 4 is at least as large as the diameter of the receiving part 7 so that the sleeve rests on the bottom of the U-shaped recess when it is inserted into the receiving part 7.

The sleeve 4 further comprises a slit 15 extending from the first end to the second end. The width of the slit 15 is sized such that when the sleeve 4 surrounds the rod 3 without any pressure force acting onto the sleeve, the slit is open and has a first width. When pressure is exerted onto the sleeve 4 via the fixation element, the sleeve is compressed and the slit has a second width smaller than the first width. The sleeve 4 is made of material which is non-flexible compared to the material of the rod. For example, the sleeve 4 is made of bio-compatible metal like stainless steel or titanium or of a stiff polymer like carbon filled PEEK or other synthetic material. The slit 15 confers elasticity to the sleeve 4 in such a manner that the sleeve 4 can be compressed thereby reducing the width of the slit. The slit 15 also allows to snap the sleeve 4 onto the rod.

On the inner wall of the sleeve 4 a plurality of circumferentially extending rib-like protrusions 16 is provided. As shown in the embodiment, the rib-like protrusions are rounded in order not to violate the surface of the rod.

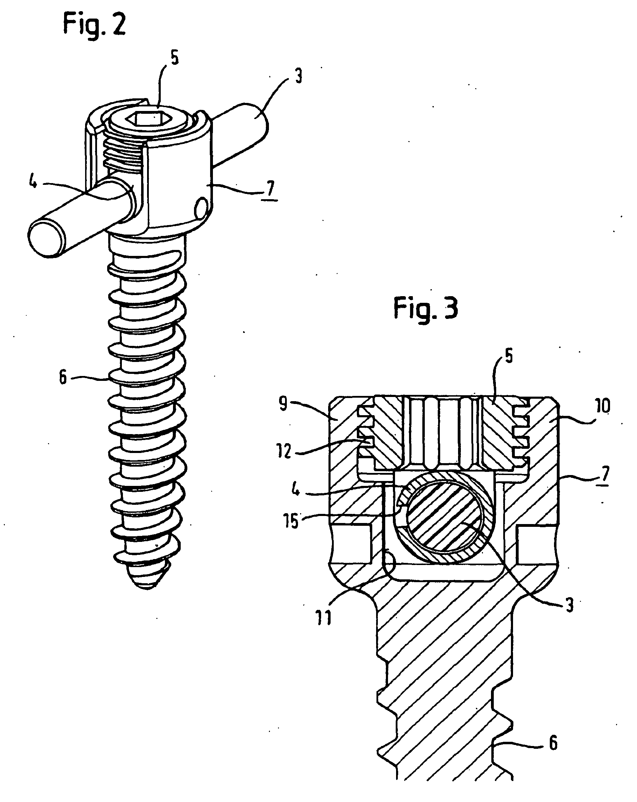

As can be seen in particular in Fig. 3, in the assembled state, the sleeve 4 is oriented on the rod in the receiving part 7 in such a way that the slit 15 faces towards one of the legs 9,10 of the receiving part, i.e. is substantially perpendicular to the screw axis.

In use, at least two bone anchoring elements 2 are screwed into adjacent vertebrae or bone parts. Then, the flexible rod 3 is provided with sleeves 4 at a distance corresponding to the distance of the bone anchoring elements 2. The rod 3 and a plurality of sleeves can either be preassembled or can be placed onto the rod during surgery. The sleeves 4 are displaceable along the rod axis as long as no pressure is exerted onto the sleeves. Hence, the surgeon can adjust the position of the sleeves on the rod.

Then, the rod together with the sleeves is inserted into the receiving parts of the respective bone anchoring elements and the fixation screw 5 is screwed in between the legs. When the fixation screw is tightened, it is pressed onto the sleeve as shown in Fig. 3, thereby compressing the sleeve 4 so that the width of slit 15 decreases. The materials of the rod 3 begins to flow, allowing the rib-like protrusions 16 to press onto the rod which provides a form-fit connection between the rod 3 and the inner wall of the sleeve 4. The integral structure of the rod is not violated. Simultaneously, the sleeve 4 is pressed onto the bottom of the U-shaped recess so that the rod is firmly held in the receiving part 7.

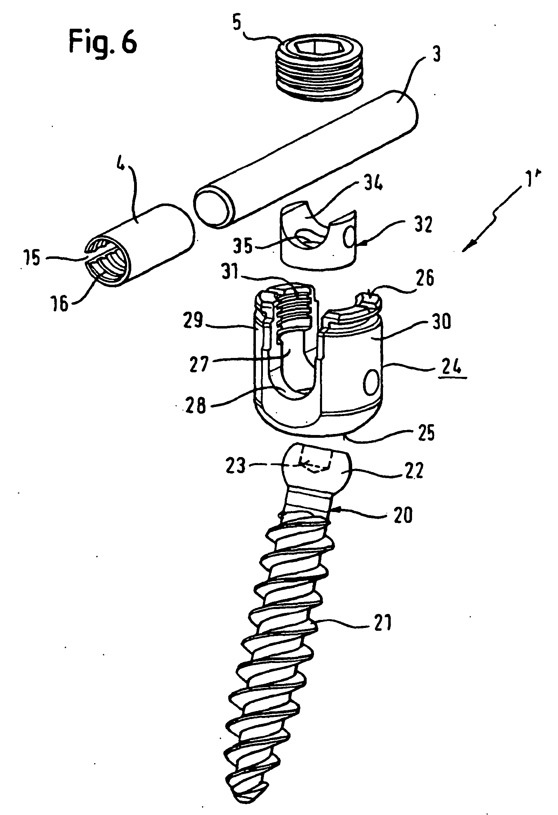

A second embodiment will now be described with reference to Figs. 6 to 8. The second embodiment of the bone anchoring device 1' comprises a polyaxial bone screw comprising a bone anchoring element 20 with a threaded shaft 21 and a head 22 which has the shape of a segment of a sphere. The head 22 has a recess 23 on its side facing away from the threaded shaft 21 in order to allow screwing-in of bone anchoring element.

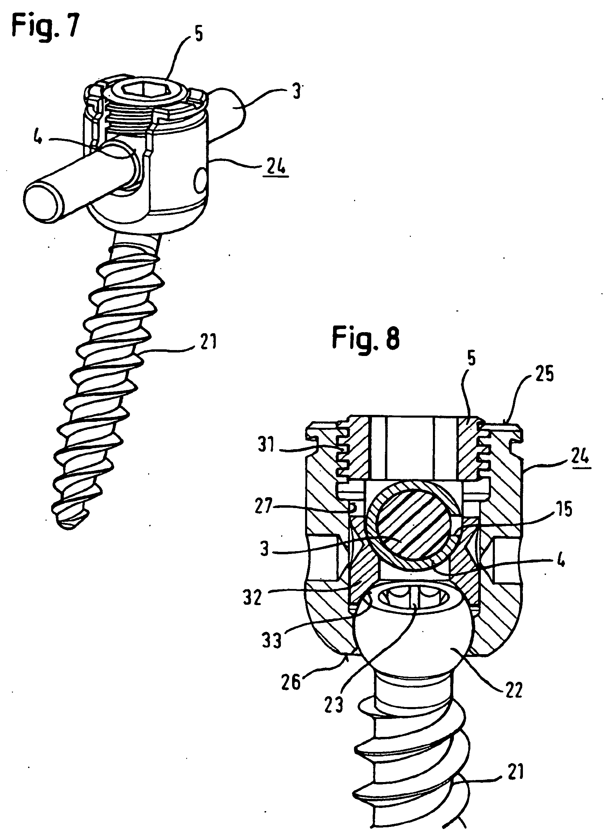

The bone anchoring device 1' further comprises a receiving part 24 having a first end 25 and a second end 26 opposite to the first end and a coaxial bore 27 extending from the second end 26 to the first end 25 and as shown in Fig. 8 tapering towards the second end 25 to provide a seat for the head 22. The receiving part 24 further has a substantially U-shaped recess 28 which provides a channel for receiving the rod 3. By means of the U-shaped recess 28 two free legs 29, 30 are formed which comprise an inner thread 31 cooperating with the fixation element 5.

A pressure element 32 is provided which has a generally cylindrical construction with an outer diameter, which is only slightly smaller than the inner diameter of the bore 27 to allow the pressure element 32 to be introduced into the receiving part and to be moved therein in the axial direction. On its lower side facing towards the second end 25, the pressure element 32 comprises a spherical recess 33 the radius of which corresponds to the radius of the spherical head 22 of the bone anchoring element. On the opposite side the pressure element has a cylindrical recess 34 extending transversely to the axis of the coaxial bore 27. The lateral diameter of this recess is sized such that the rod 3 with sleeve 4 can be inserted into the recess and guided therein. The depth of the cylindrical recess 34 is sized such that in an assembled state when the rod 3 with the sleeve 4 is inserted and pressed down by the fixation screw 5, the pressure element 34 exerts a pressure onto the head 22. In addition, the pressure element 32 has a coaxial bore 35 for guiding a tool therethrough.

In use the bone anchoring device can be pre-assembled in such a manner that the bone anchoring element 20 is pivotably held in the receiving part 24 and the pressure element 32 is inserted so that the cylindrical recess 34 is coaxial with the U-shaped recess 28 of the receiving part. At least two bone anchoring elements are screwed into adjacent vertebrae or bone parts and then the rod 3 with the sleeve 4 placed thereon is inserted into the U-shaped recess. The sleeve 4 is as in the first embodiment oriented in such a way that the slit 15 is oriented laterally. When the fixation screw 5 is inserted into the receiving part and tightened, it exerts pressure onto the sleeve, thereby compressing the sleeve to clamp the rod. The remaining pressure.force is transferred via the sleeve to the pressure element which in turn presses onto the head 22 of the bone anchoring element to fix the head in a previously adjusted angular relationship with respect to the receiving part 24. Like in the first embodiment the rib-like protrusions 16 of the inner wall of the sleeve lead to a partially form-fit connection between the flexible rod 3 and the sleeve 4 which firmly clamps the rod and prevents a flowing of the material of the rod in a direction along the rod axis.

The inner wall of the sleeve can have a roughened surface to enhance gripping of the flexible rod. The roughened surface can be provided in addition to the protrusions.

The wall thickness and the inner diameter of the sleeve 4 can vary so that different rods with different diameters can be placed into a receiving part of a predetermined size.

Instead of the described polyaxial screw, which is a polyaxial screw of the top-loader type, other kinds of polyaxial screws can be used such as bottom-loader types. Instead of the single fixation screw 5 any other fixation element can be used including dual-part fixation elements, outer nuts, etc. Instead of a threaded shaft, any other types of shafts like a hook, a cannulated shaft with openings, a shaft with barb elements, etc. can be used.

## Claims

1. Bone anchoring device comprising
a bone anchoring section (6,21) for anchoring in a bone or a vertebrae,
a receiving part (7, 24) for receiving a rod (3),
a rod (3),
a fixation element (5) for fixing the rod (3) in the receiving part (7, 24), wherein
a sleeve (4) is provided around the rod (3) in the receiving part, wherein
the sleeve (4) comprises a slit (15) having a width extending through its wall from one end to the opposite end, the width of the slit (15) being reduced when the sleeve (4) surrounds the rod (3) and a pressure force is exerted by the fixation element (5) onto the sleeve (4), wherein
the rod is at least partly made of a flexible elastomer material and in that the sleeve (4) is made of a material which is less flexible than the material of the rod (3),
and wherein
the inner wall of the sleeve (4) comprises protrusions (16) **characterized in that**,
the protrusions (16) are shaped as circumferential ribs.

2. Bone anchoring device according to claim 1, wherein the inner diameter of the sleeve (4) is slightly larger than the outer diameter of the rod (3).

3. Bone anchoring device according to claims 1 or 2, wherein the sleeve (4) is made of metal or another rigid material.

4. Bone anchoring device according to one of claims 1 to 3, wherein the receiving part (7, 24) is substantially cylindrical and comprises a U-shaped recess (8, 28) forming two free legs (9, 10; 29, 30).

5. Bone anchoring device according to one of claims 1 to 4, wherein the fixation element (5) is a locking screw.

6. Bone anchoring device according to one of claims 1 to 5, wherein the bone anchoring section (6) and the receiving part (7) are connected monoaxially.

7. Bone anchoring device according to one of claims 1 to 6, wherein the bone anchoring section (21) and the receiving part (24) are pivotably connected.

8. Bone anchoring device according to claim 1, wherein the slit (15) is open in a direction which is perpendicular to the direction of the pressure force exerted by the fixation element (5).

9. Bone anchoring device according to one of claims 1 to 8, wherein the inner surface of the sleeve comprises a surface roughness.

## Patentansprüche

1. Eine Knochenverankerungsvorrichtung, umfassend:
einen Knochenverankerungsabschnitt (6, 21) zum Verankern in einem Knochen oder einem Wirbel,
ein Aufnahmeteil (7, 24) zum Aufnehmen eines Stabs (3),
einen Stab (3),
ein Fixationselement (5) zum Fixieren des Stabs (3) in dem Aufnahmeteil (7, 24), wobei eine Hülse (4) um den Stab (3) herum in dem Aufnahmeteil vorgesehen ist,
wobei die Hülse (4) einen Schlitz (15) mit einer Breite umfasst, der sich durch deren Wand hindurch von einem Ende zum gegenüberliegenden Ende erstreckt, wobei die Breite des Schlitzes (15) reduziert ist, wenn die Hülse (4) den Stab (3) umschließt und eine Druckkraft durch das Fixationselement (5) auf die Hülse (4) ausgeübt wird,
wobei der Stab (3) zumindest teilweise aus einem flexiblen Elastomermaterial gebildet ist, und die Hülse (4) aus einem Material gebildet ist, welches weniger flexibel als das Material des Stabs (3) ist, und wobei die innere Wand der Hülse (4) Vorsprünge (16) umfasst,
**dadurch gekennzeichnet, dass** die Vorsprünge (16) als ringsumlaufende Rippen geformt sind.

2. Die Knochenverankerungsvorrichtung gemäß Anspruch 1,
wobei der Innendurchmesser der Hülse (4) geringfügig größer als der Außendurchmesser des Stabs (3) ist.

3. Die Knochenverankerungsvorrichtung gemäß einem der Ansprüche 1 oder 2,
wobei die Hülse (4) aus einem Metall oder einem anderen starren Material gebildet ist.

4. Die Knochenverankerungsvorrichtung gemäß einem der Ansprüche 1 bis 3,
wobei das Aufnahmeteil (7, 24) im Wesentlichen zylindrisch ist und eine U-förmige Ausnehmung (8, 28) umfasst, die zwei freie Schenkel (9, 10; 29, 30) ausbildet.

5. Die Knochenverankerungsvorrichtung gemäß einem der Ansprüche 1 bis 4,
wobei das Fixationselement (5) eine Sperrschraube ist.

6. Die Knochenverankerungsvorrichtung gemäß einem der Ansprüche 1 bis 5,
wobei der Knochenverankerungsabschnitt (6) und das Aufnahmeteil (7) monoaxial miteinander verbunden sind.

7. Die Knochenverankerungsvorrichtung gemäß einem der Ansprüche 1 bis 6,
wobei der Knochenverankerungsabschnitt (21) und das Aufnahmeteil (24) schwenkbar miteinander verbunden sind.

8. Die Knochenverankerungsvorrichtung gemäß einem Anspruch 1,
wobei der Schlitz (15) in einer Richtung offen ist, die senkrecht zur Richtung der Druckkraft ist, die durch das Fixationselement (5) ausgeübt wird.

9. Die Knochenverankerungsvorrichtung gemäß einem der Ansprüche 1 bis 8,
wobei die Innenoberfläche der Hülse eine Oberflächenrauigkeit aufweist.

## Revendications

1. Dispositif d'ancrage osseux comprenant
une section (6, 21) d'ancrage osseux à ancrer dans un os ou une vertèbre,
une partie de réception (7, 24) pour recevoir une tige (3),
une tige (3),
un élément de fixation (5) pour fixer la tige (3) dans la partie de réception (7, 24), où
un manchon (4) est pourvu autour de la tige (3) dans la partie de réception, où
le manchon (4) comprend une fente (15) ayant une largeur s'étendant à travers sa paroi d'une extrémité à l'extrémité opposée, la largeur de la fente (15) étant réduite lorsque le manchon (4) entoure la tige (3) et une force de pression est exercée par l'élément de fixation (5) sur le manchon (4), où
la tige est au moins partiellement faite d'un matériau en élastomère flexible et le manchon (4) est fait d'un matériau qui est moins flexible que le matériau de la tige (3), et où
la paroi interne du manchon (4) comprend des protubérances (16), **caractérisé en ce que**
les protubérances (16) sont formées comme des nervures circonférentielles.

2. Dispositif d'ancrage osseux selon la revendication 1, dans lequel le diamètre interne du manchon (4) est légèrement plus grand que le diamètre externe de la tige (3) .

3. Dispositif d'ancrage osseux selon la revendication 1 ou 2, dans lequel le manchon (4) est fait d'un métal ou d'autre matériau rigide.

4. Dispositif d'ancrage osseux selon l'une des revendications 1 à 3, dans lequel la partie de réception (7, 24) est essentiellement cylindrique et comprend une cavité en forme de U (8,28) formant deux pieds libres (9, 10 ; 29, 30).

5. Dispositif d'ancrage osseux selon l'une des revendications 1 à 4, dans lequel l'élément de fixation (5) est une vis de verrouillage.

6. Dispositif d'ancrage osseux selon l'une des revendications 1 à 5, dans lequel la section (6) d'ancrage osseux et la partie de réception (7) sont reliées de manière monoaxiale.

7. Dispositif d'ancrage osseux selon l'une des revendications 1 à 6, dans lequel la section (21) d'ancrage osseux et la partie réception (24) sont reliées en pivotement.

8. Dispositif d'ancrage osseux selon la revendication 1, dans lequel la fente (15) est ouverte dans une direction qui est perpendiculaire à la direction de la force de pression exercée par l'élément de fixation (5).

9. Dispositif d'ancrage osseux selon l'une des revendications 1 à 8, dans lequel la surface interne du manchon a une rugosité de surface.
